# EUROPEAN PATENT APPLICATION

(11) **EP 1 958 628 A1**
(43) Date of publication of application: **20.08.2008**
(21) Application number: 07003281.8
(22) Date of filing: 16.02.2007
(51) Int. Cl.: A61K 31/353, A61K 31/585, A61K 9/20, A61K 9/22, A61K 9/26, A61K 9/16, A61K 9/48

(54) **Combination of immediately liberated drospirenone with a modified releasing formulation containing 8-prenylnaringenin for use in oral contraception and hormone replacement therapy**

(71) Applicant: KAIROSmed GmbH, 10117 Berlin (DE)
(72) Inventor: Hümpel, Michael, 14163 Berlin (DE); Tack, Johannes, 13595 Berlin (DE)
(74) Representative: Jungblut, Bernhard Jakob

(57) **Abstract**

This invention is directed to an oral modified releasing formulation of the phytoestrogen 8-Prenylnaringenin in combination with an immediately releasingd progestin, preferably the progestin Drospirenone, and its use for combination oral contraception or hormone replacement therapy.

## Description

This invention is related to the combination of an immediately liberating formulation containing a progestin, preferably the synthetic progestin Drospirenon (DRSP), and an oral modified releasing preparation containing the estrogenic natural compound 8-Prenylnaringenin (8-PN) and their use in oral contraception in fertile women and in oral hormone replacement therapy in menopausal women.

Combination oral contraception (COC) and combination oral hormone replacement therapy (HRT) have been known for decades. Both medications generally contain a synthetic progestin and a steroidal estrogen, which is predominantly ethinyl estradiol (EE) in case of COC and estradiol (E2) or its metabolites (estrone, estriol) or pro-drugs (esters) in case of HRT. Since first introduction into the market, innovations in COC have mainly been aimed at the reduction of drug-free intervals, different absolute dose levels of active ingredients, the progestin/estrogen dose ratios during one treatment cycle, and the choice of the progestin. Respective innovations in oral HRT similarily concern reduction of active ingredient doses, dose ratios, and the choice of progestin. In addition, a continuous treatment schedule avoiding bleeding periods has been developed recently. The preponderance of the progestin renders the endometrium atrophic after several months of use with the effect that vaginal bleedings diminish over time and stop almost completely after 6 to 12 months.
While in COC the progestin is mainly responsible for the inhibition of ovulation, the pharmacological effect of the progestin in combination HRT mainly is to counteract the estrogen at the endometrial level. Consequently the daily dose of a respective progestin in HRT is about 30 % lower than its effective dose in COC.
Progestins can be divided into two chemical classes, i.e. the the 19-nor-testosterone derivatives and the so-called C-21 progestins. One pharmacological property of 19-nor-testosterone derivatives (norethisterone, levonorgestrel and others) is their partially androgenic activity leading to unwanted effects among which sebaceous skin and body weight gain are the most common ones. The so-called C-21 progestins (medroxyprogesterone acetate, cyproterone acetate and others) do not exhibit androgenic activity like natural progesterone. However, progestins from both the two chemical classes lack the anti-mineral-corticoid activity of progesterone. Recently such a progestin was found as a progestagenic metabolite of a spironolactone derivative. Drospirenone (DRSP) has been characterized as a highly potent progestin with some anti-androgenic and aldosterone antagonistic activities and thus almost perfectly mimicks the pharmacological profile of natural progesterone. The structure of DRSP is as follows:

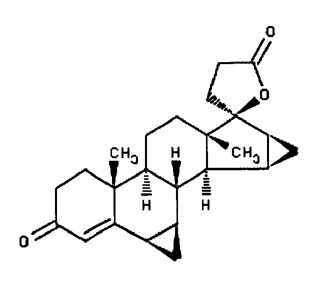

The aldosterone-antagonistic activity of DRSP were the subject of a patent application in 1976 (Schering AG). Another patent application was filed in 1977 claiming the substance as a diuretic drug. Its use as a progestin in oral contraception and gynecological indications was patented in 1980. Thus, the patent protection of the substance ran out at the same time when the drug came to the market as constituent of the COC Yasmin® (3 mg DRSP plus 30µg EE; market entry in Germany was November 2000). The respective HRT preparation (Angeliq®, DRSP 2 mg plus 1.0 mg E2) is on the market since 2005. The patent protection of Yasmin® and Angeliq® (as well as of EE dose reduced COC YAZ®) is mainly based on a technical patent claiming a stable oral formulation with acute drug release (US 6,787,531).
While in case of Yasmin® treatment schedule follows the classical approach of 21 days fixed combination active treatment followed by a 7 days drug-free interval, in case of YAZ® (DRSP 3 mg plus 20 µg EE) the fixed combination active treatment is for 24 days followed by a 4 days drug-free interval). Treatment schedule for Angeliq® is continuous.
In contrast to the large variety of progestins used in COCs or HRT preparations, their respective estrogenic component almost exclusively consists of EE (or its pro-drug mestranol) in case of COCs and E2 (or its metabolites, esters or conjugated equine estrogens (CEE)) in case of HRT. EE is a synthetic derivative of E2. By 17α-ethinylation the rate of metabolic degradation of E2 in the liver is slowed down leading to an increase of oral E2-bioavailability of about 1 - 3% of dose to roughly 40 % of dose (EE). However, despite this partial metabolic stabilization, with respect to oral bioavailability of E2 or EE large variations exist between individuals. The coefficient of variation (CV) of the mean area under the plasma level time curve (AUC) after oral 30 µg EE ranges between 75 and 94 % and accounted for 127 %, 91 %, or 70 % after oral 2, 4, or 8 mg E2, respectively. Thus, compared to DRSP figures (CV of AUC's of about 20 %) the systemic availability of the estrogenic components in COC and HRT is at much higher variance than that of the progestogenic component.
Generally, high variances in drug availability should be avoided in medical treatments. Whereas individual dose titration can solve the problem when a single drug is used, in fixed dose combination treatments with mainly counteracting pharmacological effects of both active ingredients a much higher variability in drug levels of one ingredient bears the risk of an imbalanced overall effect. Interestingly, a COC, newly introduced into the market, generally starts with a higher EE dose of ≥ 0.03 mg/d. This dose constitutes an overdose for a sub-population of women and consequently the original preparation is often followed by an EE reduced version (mostly 0.02 mg/d).
A similar situation exists in combination oral HRT. Daily oral E2 doses in various prepararions vary between 0.8 mg (equivalent to 1 mg E2 valerate; Mericomb®, Novartis) and 4 mg (Trisequens forte®, NOVO).
Although the pharmacokinetics of EE and E2 do not meet the requirements for a reliable support of an highly active drug there were only a few attempts to substitute EE in COC or E2 in HRT. One refers to the use of E2 in COC, but even high doses of 4mg/d or more did not resolve the problem of insufficient bleeding control in respective developmental COCs. This can be explained by the fact that the product of concentration and time at the estrogen receptors (ERα, ERβ) was not sufficiently high for E2 due to rapid metabolic inactivation. Most probably, E2 concentrations at the ERs were below a threshold level for many hours of the 24 hours treatment interval. A similar situation exists in HRT. The available oral combined preparations containing E2 (or its metabolites or esters) cannot support the estrogen continuously over the treatment interval but lead to highly fluctuating drug levels with sub-therapeutic concentrations for many hours a day.
In summary, there is a medical need for a substitution of EE as the estrogen in COC and of E2 in HRT. The substitute should reliably (CV of AUC < 50 %) become bioavailable after oral administration and be present at sufficiently high concentrations at the estrogen receptors (ERα, ERβ) for the total treatment interval of 24 hours.
Recently, a secondary plant metabolite was characterized as a new estrogen receptor agonist. The active ingredient, 8-Prenylnaringenin (8-PN), had been identified in plants (*Humulus lupulus L., Anaxagorea luzonensis* A. Gray) and was shown to be not only the most active agonistic secondary plant metabolite at the estrogen receptor but also a substance that exhibits tissue specificity with a much lower activity on the uterus than E2 at equivalent bone protective doses. 8-PN (5,7-Dihydroxy-2-(4-hydroxyphenyl)-8-(3-methylbut-2-enyl)-chroman-4-on) has the structure given below.
The 4-hydroxyphenyl group may either be in the 2S(-) or the 2R (+) position.
8-PN was characterized as a pure estrogen receptor agonist devoid of binding or activation of the PR, AR, or GR and without any anti-estrogenic activity.

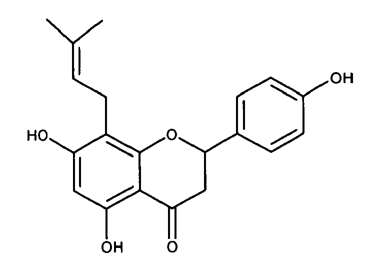

Interestingly, 8-PN is mainly inactivated by conjugation. 8-PN conjugates are excreted predominantly with the bile fluid, deconjugated in the duodenum and intestinum after bile fluid secretion and then re-absorbed as unchanged drug. This process is known as entero-hepatic re-circulation (EHC) and constitutes the basis for a multiple re-use of the drug. 8-PN exhibits a stable systemic availability with a CV of 42 or 29 % of mean AUC at oral doses of 250 or 750 mg, respectively. In addition, due to a surprisingly low extent of metabolism by CYP isoenzymes 8-PN shows a stable systemic availability irrespective of the mode of administration, i.e. acute or modified releasing. Thus, 8-PN is a suitable drug for use in modified releasing formulations and - by this - can substitute EE in COC as well as E2 in HRT. Respective preparations are composed of an immediately releasing part for the progestin and of a modified releasing part for 8-PN.

The problem, therefore, is to provide an oral formulations for a suitable so-called C21-progestin, preferably DRSP, and a substitute for EE (mestranol) in case of COC or for E2 (metabolites or esters) in case of HRT, preferably 8-PN, which immediately releases the progestin but continuously distributes 8-PN for the gastro-intestinal transit time of generally 12 - 16 hours. Preparations have preferably to be administered once a day, preferably in the evening.
This problem was solved by two component preparation containing a solid oral immediate releasing part for the progestin DRSP and a solid oral modified releasing part for 8-PN.
The immediate releasing part contains excipients to form a granulate for compression like fillers, binders, disintegrators and lubricants in addition to DRSP or to form a direct compression mass like microcristalline cellullose, and/or other fillers and disintegrators and lubricants in addition to DRSP. The fillers are preferably chosen from the group of monosaccharides like e. g. lactose or sugar alcohols e.g. mannitol; binders are preferably chosen from celluloseethers like e. g. hydroxypropylcellulose or polyvinylpyrrolidones like e. g. Kollidone 25000; disintegrators are preferably chosen from starch or modified starch like e.g. maize starch; lubricants are usually salts of fatty acids or mixtures with them, preferably magnesium stearate or calcium behenate.

The modified releasing part contains a polymeric matrix, a buffer substance and one or more excipients in addition to 8-PN. Preferably, the buffer substance is an alkaline substance like e. g. magnesium oxide, magnesium hydroxide, dihydroxyaluminum aminoacetate, magnesium carbonate, calcium carbonate, sodium ascorbate, magnesium trisilicate, dihydroxyaluminum sodium carbonate, aluminum hydroxide, sodium citrate, potassium phosphate, sodium bicarbonate, disodium hydrogenphosphate or some combinations thereof. Preferably, the particle size of the formulation components is in the range of 0.1 - 750 µm. Most preferably, it is in the range of 2 - 400 µm. The polymer matrix is preferably chosen from the group consisting of: cellulose derivatives, acrylic derivatives, vinyl polymers, polyacrylates, polycarbonates, polyethers, polystyrenes polyanhydrides, polyesters, polyorthoesters, polysaccharides and natural polymers. Most preferably, the polymer matrix consists of water soluble polyvinylpyrrolidone and/or water insoluble polyvinylacetate. In a preferred embodiment the oral modified releasing formulation is coated with a polymeric coat.

Excipients may be chosen from the group consisting of lactose, calcium phosphate, manitol and starch. Preferably, an additional excipient is microcrystalline cellulose.

The solid oral modified releasing formulations of this invention show a pH-independent drug release in vitro. This is important as the pH varies considerably in the gastro-intestinal tract and continuous releasing should be achieved independent of the pH. However, the solubility of 8-PN is pH-dependent. The compound shows higher solubility at higher pH-values whereas the solubility of the compound is low at lower pH-values. The low acid solubility property of 8-PN results *in vitro* in slow drug dissolution at pH 1, whereas the dissolution is fast at higher pH-values such as pH 6.8. The resulting dissolution profiles are different at different pH-values. This problem is solved by the solid oral modified releasing formulation of this invention.

The solid oral modified releasing formulation of this invention solves the problem of a continuous distribution of 8-PN almost over 24 h. This is a combined effect of the pharmacokinetic profile of 8-PN (high metabolic stability versus CYP isoenzymes, EHC) which is drastically different from those of steroidal estrogens and the solid oral modified releasing formulation.

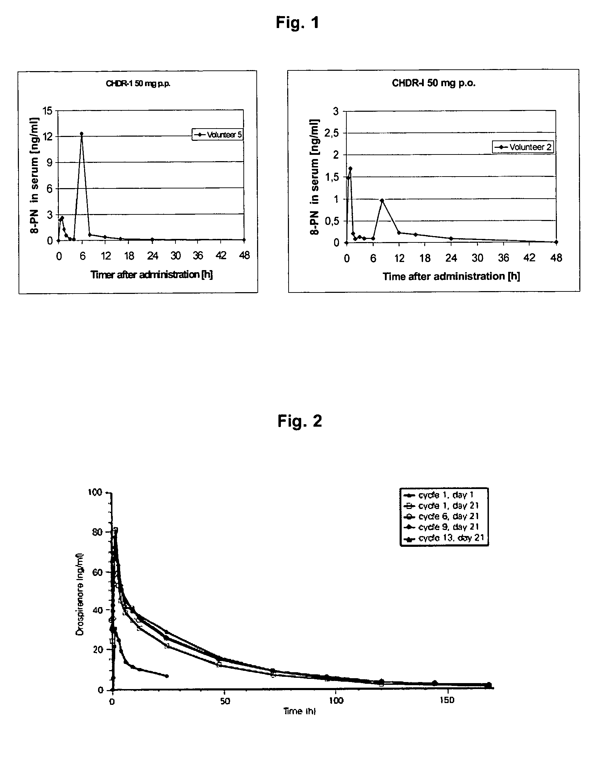

The pharmacokinetic profile of 8-PN is characterized by a complete oral absorption, a low degree of metabolisation (less than 60 % of dose) and a high pre-systemic elimination (first-liver-pass excretion; about 55 % of dose). The high and unexpected pre-systemic elimination leads to a collection of substantial dose parts in the bile fluid from which these dose parts are secreted into the duodenum when gastric signaling occurs with a meal uptake. Respective dose parts are then absorbed again and reach systemic circulation. Examples for this effect (drug serum levels) are shown in Fig. 1. Data were taken from the clinical Phase la study and represent two volunteers of the lowest dose group (50 mg 8-PN).

Taking the total area under the curve as a measure for 8-PN systemic availability, the percentage of the area under the second peak (generated by reabsorption of the pre-systemically eliminated and then biliary secreted dose parts) can be used to estimate those dose parts which undergo pre-systemic elimination after oral dosing. On an average of six women investigated this figure is 54 ± 20 %.

The invention is to combine this unexpected pharmacokinetic property of 8-Prenylnaringenin with a modified releasing formulation, which can release the drug at an almost constant rate for 8 - 10 hours. The drug releasing from the solid oral modified releasing formulation according to this invention is preferably close to or perfect zero order kinetics. This oral modified releasing formulation is preferably taken in the evening (after dinner or before bed-time). During nighttime 8-PN is released at almost constant rate leading to flat drug serum levels and a collection of substantial dose parts in the bile fluid which - after reabsorption - account for more than half of total systemically available dose.

The next day these dose parts are secreted into the duodenum when the first meal (breakfast or lunch) is taken. Re-absorption gives rise to elevated 8-Prenylnaringenin serum levels over the first half of the day, which - at a lower level - repeats with dinner in the evening. Overall, the total systemically available dose is distributed over 24 hours avoiding unnecessary high peaks and ensuring effective drug concentrations in target tissues. For use in COC the anticipated daily dose is between 50 and 250 mg, preferably between 75 and 150 mg. For use in HRT the anticipated daily dose is between 25 and 250 mg, preferably between 50 and 150 mg The oral modified releasing formulation is preferably taken 6 - 12 hours, more preferably 8 - 12 hours before having a meal.

The pharmacokinetic profile of DRSP is characterized by complete oral absorption, an absolute oral bioavailability of 76 %, and a disposition half-life of 31 hours. The apparent volume of distribution was estimated to 4 L/kg. After oral intake of 3 mg DRSP maximum serum levels of 37 ng/ml were reached at 1.7 hours. AUC₀₋₂₄ₕ was 288 ngxh/ml. Following daily repeated administration the maximum serum level increased to about 80 ng/ml and AUC to about 920 ngxh/ml. Increases are the result of drug accumulation at daily treatment and a half-life of 31 hours. Figure 2 shows time course and degree of accumulation.
DRSP does not bind to SHBG and does not suppress EE induced SHBG or CBG serum levels. DRSP is only slightly metabolized, mainly by CYP3A4.
DRSP shows a 2 to 3 fold accumulation after acute drug release from the film-coated tablet. Identical mean PK parameters will result with any immediately releasing formulation when complete DRSP absorption is ensured and the daily treatment schedule is not changed.

This invention provides a method of hormonal contraception and hormone replacement therapy by administering a two component preparation consisting of a solid oral modified releasing part containing 8-PN and an immediately releasing part containing DRSP to the patient once daily.

Still another aspect, this invention provides a method of maintaining therapeutically effective levels of 8-PN and DRSP in human plasma or serum by administering an 8-PN containing oral modified releasing part combined with an immediately releasing DRSP part once daily.

The method includes administering an oral modified releasing formulation part including from about 5 to about 80 % by weight 8-PN and an immediately releasing part including less than 10 % by weight DRSP in one multiple unit dosage form per dose to women. In case of oral contraception 8-PN plasma levels from about 0.5 to about 5 ng 8-PN/ml are to be maintained for at least 24 hours wherein the dose is administered once a day. The daily dose of co-administered DRSP is 3 mg leading to mean maximum drug levels of about 75-85 ng/ml at steady state.
In case of HRT 8-PN plasma levels from about 0.5 to about 5 ng 8-PN/ml are to be maintained for at least 24 hours wherein the dose is administered once a day. The daily dose of co-administered DRSP is 1 mg leading to mean maximum drug levels of about 25 - 30 ng/ml at steady state.
The effective dose of 8-PN as well as of DRSP may be contained in one formulation unit or alternatively in two separate formulation parts and then preferably packed and sealed together for example in one blister mould. The dose of 8-PN as well of DRSP can be kept constant or can be varied during the active treatment over the 21 days or 24 days treatment period.
For example a blister pack for use in combination contraception may be designed that the first six blister moulds contain a DRSP formulation described in this invention dosed between 0.25 - 0.5 mg each together with a 8-PN formulation described in this invention dosed for example with 100 mg each and both actives are either incorporated in one single hardcapsule or in two separate hardcapsules. The second five moulds may contain in the way described above a suited DRSP formulation dosed between 1.5 to 2.0 mg each together with 8-PN formulations dosed with 150 mg each and the final ten moulds contain a DRSP formulation as described above containing 3.0 mg DRSP each together with 100 mg 8-PN each. Other combination may be useful to support efficacy and cycle control of the preparation.

The formulation units of this invention may be either in the form of single unit dosages, e.g. tablets, or in the form of multiple unit dosages, e.g. granulates, pellets or mini-tablets. These multiple unit dosages may be filled in gelatine capsules or compressed to tablets.

The single unit dosages may be produced by powder blending and direct compression into tablets or by powder blending, granulation and compression into tablets. The tablets may be coated by a film.

Multiple unit dosage may be produced by extrusion/spheronization, by layering technique, by rotor granulation, by powder blending and direct compression into mini tablets, by powder blending, granulation and compression into mini tablets, by powder blending, direct compression into mini tablets and film coating or by powder blending, granulation, compression into mini tablets and film coating.

8-Prenylnaringenin can be produced by the method described in WO 2005/037816. Drospirenone can be produced by the method described in US 6,933,395.

### Description of the figures

Fig. 1 shows drug serum levels following a single oral dose of 50 mg 8-Prenylnaringenin in two postmenopausal women; 8-Prenylnaringenin was administered as a 1:1 mixture with lactose (gelatine capsule) at fasted state in the morning, lunch was served 6 hours later. Re-increases in drug serum levels show re-absorption of dose parts collected in the bile fluid and subsequently secreted triggered by lunch.
Fig. 2 shows DRSP serum levels after oral administration of 3 mg DRSP combined with 0.03 mg EE in 13 women. The COC was taken for 13 cycles at a 21 days/7 days regimen. Samples were drawn on day 1 of cycle 1 and on day 21 of cycles 1, 6, 9, and 13. The figure was taken from Blode H, Wuttke W, Loock W, Heithecker R (2000): The European Journal of Contraception and Reproductive Care, 5; 256-264

### Examples:

### Example 1

### Production of Mini-Matrix Tablets containing 8-PN by Means of Direct Tableting

2.333 mg of 8-Prenylnaringenin
1.000 mg of Kollidon SR®
1.169 mg of magnesium oxide
0.823 mg of lactose
1.500 mg microcrystalline cellulose
0.070 mg of highly dispersed silicon dioxide
0.105 mg of magnesium stearate

8-Prenylnaringenin, Kollidon SR®, magnesium oxide, lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press. The releasing from these mini-tablets is measured by means of the method that is mentioned in Example 3.

### Example 2

### Production of Mini-Matrix Tablets containing 8-PN by Means of Direct Tableting

2.333 mg of 8-Prenylnaringenin
1.000 mg of Kollidon SR®
1.169 mg of magnesium hydroxide
0.823 mg of lactose
1.500 mg microcrystalline cellulose
0.070 mg of highly dispersed silicon dioxide
0.105 mg of magnesium stearate

8-Prenylnaringenin, Kollidon SR®, magnesium hydroxide, lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press. The releasing from these mini-tablets is measured by means of the method that is mentioned in Example 3.

### Example 3

### Measurement of the Release of 8-Prenylnaringenin

Measurement of the active ingredient release from mini-matrix tablets is carried out according to a one-compartment method (basket apparatus), as described in U.S. Pharmacopeia USP XXV. The release of 8-Prenylnaringenin was examined in phosphate buffer solution, pH 6.8 (composition, see USP XXV) or in 0.1 N HCl. Ten percent (w/w) hydroxypropyl-β-cyclodextrine were added in order to achieve sink conditions and primarily control the drug release by the dosage form.

### Example 4

### Production of DRSP containing immediate release parts by direct compression

0,300 mg of DRSP
1.150 mg of lactose monohydrate
4.500 mg microcrystalline cellulose
1.000 mg maize starch
0.050 mg of magnesium stearate

DRSP, lactose and microcrystalline cellulose and maize starch are sieved individually and mixed in a turbula mixer for 10 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release of DRSP from these mini-tablets is measured by means of the method that is mentioned in Example 6.

### Example 5

### Production of DRSP containing immediate release parts by granulation and compression

0,300 mg of DRSP
4.650 mg of lactose monohydrate
0.500 mg polyvinylpyrrolidon (Kollidone 25000)
1.500 mg maize starch
0.050 mg of magnesium stearate
(processing excipient to be removed during manufactuiring: Demineralized water)

DRSP, lactosemonohydrate and polyvinylpyrrolidon and maize starch are sieved individually and mixed in a shear-mixer for 10 minutes. Demineralized water is added to the blend in an amount to form granules by further mixing. The granules are sieved and subsequently dried to remove the water. Magnesium stearate, sieved, is spread on, and all components are mixed for another 30 seconds. Tableting of the granulate into mini-matrix tablets is carried out by means of an eccentric tablet press or a rotary tablet press.
The release of DRSP from these mini-tablets is measured by means of the method that is mentioned in Example 6.

### Example 6

### Measurement of the release of DRSP

Measurement of the active ingredient release from mini-matrix tablets is carried out according to a one-compartment method (basket apparatus), as described in U.S. Pharmacopeia USP XXV. The release of DRSP was examined in phosphate buffer solution, pH 6.8 (composition, see USP XXV) or in 0.1 N HCI. Ten percent (w/w) hydroxypropyl-ß-cyclodextrine were added in order to achieve sink conditions for the active ingredient and primarily control the drug release by the dosage form.

## Claims

1. Two component oral preparation containing a modified releasing 8-PN part and an immediately DRSP releasing part and where the modified releasing part contains a polymeric matrix, a buffer substance and one or more excipients.

2. Two component oral preparation containing a modified releasing 8-PN part and an immediately DRSP releasing part and where the modified releasing part contains a polymeric matrix, a buffer substance and one or more excipients and where the particle size of the compounds is in the range of 0.1 - 750 µm.

3. Two component oral preparation containing a modified releasing 8-PN part and an immediately DRSP releasing part according to claim 1 or 2 and where the effective dose of 8-PN as well as of DRSP may be contained in one formulation unit or alternatively in two separate formulation parts and preferably packed and sealed together for example in one blister mould.

4. Two component oral preparation containing a modified releasing 8-PN part and an immediately DRSP releasing part according to claim 1, 2 or 3 wherein the buffer substance is an alkaline substance.

5. Two component oral preparation containing a modified releasing 8-PN part and an immediately DRSP releasing part according to claims 1 - 4 wherein the modified releasing part is coated with a polymeric coat that affects the dissolution of 8-PN.

6. Two component oral preparation containing a modified releasing 8-PN part and an immediately DRSP releasing part according to claims 1 - 5 wherein the polymer matrix is chosen from the group of the following materials: cellulose derivatives, acrylic derivatives, vinyl polymers, polyacrylates, polycarbonates, polyethers, polystyrenes polyanhydrides, polyesters, polyorthoesters, polysaccharides and natural polymers.

7. Two component oral preparation containing a modified releasing 8-PN part and an immediately DRSP releasing part according to claims 1 - 5 wherein the polymer matrix is chosen from water soluble polyvinylpyrrolidone and water insoluble polyvinylacetate.

8. Two component oral preparation containing a modified releasing 8-PN part and an immediately DRSP releasing part according to claims 1 - 7 wherein the buffer substance is magnesium oxide, magnesium hydroxide, dihydroxyaluminum aminoacetate, magnesium carbonate, calcium carbonate, sodium ascorbate, magnesium trisilicate, dihydroxyaluminum sodium carbonate, aluminum hydroxide, sodium citrate, potassium phosphate, sodium bicarbonate, disodium hydrogenphosphate or some combinations thereof.

9. Two component oral preparation containing a modified releasing 8-PN part and an immediately DRSP releasing part according to claims 1 - 8 wherein the preparation contains an additional lubricant.

10. Two component oral preparation containing a modified releasing 8-PN part and an immediately DRSP releasing part according to claims 1 - 9 wherein the excipient is lactose, polyvinylpyrrolidone, calcium phosphate, manitol, starch or modified starch.

11. Two component oral preparation containing a modified releasing 8-PN part and an immediately DRSP releasing part according to claims 1 - 10 wherein the preparation contains microcrystalline cellulose as an additional excipient.

12. Two component oral preparation containing a modified releasing 8-PN part and an immediately DRSP releasing part according to claims 1 - 11 wherein the preparation contains silicon dioxide as flow promoter.

13. Two component oral preparation containing a modified releasing 8-PN part and an immediately DRSP releasing part according to claims 1 - 12 wherein the particle size distribution of the powder mixtures is in a range between 2 - 400 µm.

14. Two component oral preparation containing a modified releasing 8-PN part and an immediately DRSP releasing part according to claims 1-13 for the production of a medicament for the combination hormonal contraception.

15. Two component oral preparation containing a modified releasing 8-PN part and an immediately DRSP releasing part according to claims 1-13 for the production of a medicament for the combination hormone replacement therapy.

16. Two component oral preparation containing a modified releasing 8-PN part and an immediately DRSP releasing part according to claims 1-15 where the preferred daytime of ingestion is the evening or bedtime.

17. Two component oral preparation containing a modified releasing 8-PN part and an immediately DRSP releasing part according to claims 1-14 and 16 where the active oral contraceptive treatment phase is between 21 and 25 days.

18. Two component oral preparation containing a modified releasing 8-PN part and an immediately DRSP releasing part according to claims 1-14 and 16 where the active oral contraceptive treatment phase is between 21 and 25 days and where varying doses of DRSP and 8-PN are incorporated in the respective formulation parts during the active treatment period.

19. Two component oral preparation containing a modified releasing 8-PN part and an immediately DRSP releasing part according to claims 1-13 and 15-16 where the active oral hormone replacement therapy treatment phase is between 21 days and continuous.

20. Two component oral preparation containing a modified releasing 8-PN part and an immediately DRSP releasing part according to claims 1-19 where 8-Prenylnaringenin is replaced by any other estrogenic compound or any derivative of 8-Prenylnaringenin.

21. Two component oral preparation containing a modified releasing 8-PN part and an immediately DRSP releasing part according to claims 1-20 where Drospirenone is replaced by any other progestin.
